# EUROPÄISCHE PATENTANMELDUNG

(11) **EP 3 978 920 A1**
(43) Veröffentlichungstag der Anmeldung: **06.04.2022**
(21) Anmeldenummer: 20199604.8
(22) Anmeldetag: 01.10.2020
(51) Int. Cl.: G01N 33/28, G01N 22/04

(54) **AUTOMATISIERTE BESTIMMUNG VON VERÄNDERUNGEN IN FLÜSSIGEN STOFFEN MIT EINEM MIKROWELLEN HOHLRAUMRESONATOR**

(71) Anmelder: Siemens Aktiengesellschaft, 80333 München (DE)
(72) Erfinder: Schreiter, Matthias, 81379 München (DE)

(57) **Zusammenfassung**

Die Erfindung gibt ein automatisiertes Verfahren zur Bestimmung der Veränderung eines flüssigen Stoffs (2) zwischen einem ersten Zeitpunkt (T1) und einem zweiten Zeitpunkt (T2) bei einer gemessenen Betriebstemperatur (OT) an,
- wobei ein repräsentativer Teil eines Transmissionsspektrums (TS) des in einem Mikrowellen Hohlraumresonator (1) eingebrachten Stoffs (2) zu dem zweiten Zeitpunkt (T2) ermittelt wird und
- wobei der ermittelte repräsentative Teil des Transmissionsspektrums (TS) mit dem vorab zu dem ersten Zeitpunkt (T1) bei der gleichen Betriebstemperatur (OT) ermittelten repräsentativen Teil eines Referenz-Transmissionsspektrums (RTS) eines gleichen, aber unberührten und/oder unveränderten Stoffs (2) verglichen wird und
- wobei eine Abweichung der beiden repräsentativen Teile voneinander ermittelt wird. Die Erfindung gibt ebenfalls eine zugehörige Vorrichtung an.

## Beschreibung

### GEBIET DER ERFINDUNG

Die Erfindung betrifft ein automatisiertes Verfahren und eine Vorrichtung zur Bestimmung der Veränderung eines flüssigen Stoffs zwischen einem ersten Zeitpunkt und einem zweiten Zeitpunkt bei einer gemessenen Betriebstemperatur.

### HINTERGRUND DER ERFINDUNG

Im Rahmen der Digitalisierung in der Industrie gewinnen Online-Überwachungssysteme zunehmende Bedeutung zur Abbildung des Ist-Zustands einer technischen Komponente oder eines Systems mit dem Ziel einer signifikanten Kostenersparnis durch Früherkennung von Parameterabweichungen und Vermeidung größerer materieller Schäden bzw. Ausfälle (wird auch als "Predictive Maintenance" bezeichnet).

Die Herausforderung an derartige Systeme ist, dass sie zuverlässig, langzeitstabil und möglichst wartungsfrei arbeiten. Aufgrund des dezentralen Einsatzes sind in der Regel kostengünstige Lösungen Voraussetzung für die Umsetzbarkeit. Eine derartige Lösung stellt zum Beispiel ein Hohlraumresonator dar, der es beispielsweise ermöglicht, Feuchte in Ölen in sehr geringen Konzentrationen über eine Änderung der dielektrischen Eigenschaften und damit u.a. des Transmissionsspektrums (s21, Vorwärtstransmission vom Eingang 1 zum Ausgang 2) in der Umgebung einer oder mehrerer Resonanzmoden des Resonators nachzuweisen.

Der Vorteil eines Sensors mit einem Hohlraumresonators liegt in seiner einfachen, robusten Bauweise und Langzeitstabilität. Problematisch ist allerdings die starke Temperaturabhängigkeit des Transmissionsspektrums aufgrund von Längenausdehnungen des Resonators, der Temperaturabhängigkeit der Elektronik als auch der temperaturabhängigen dielektrischen Eigenschaften des Analyten, wie zum Beispiel Öl.

Die Anwendung von Hohlraumresonatoren zur Bestimmung von Feuchte in Ölen ist Stand der Technik. Kommerzielle Anbieter vertreiben Sensorlösungen auf Basis von Hohlraumresonatoren beispielsweise für die Messungen von Feuchte in Schüttgut. In der Druckschrift Hayder Hamzah et al, "A Compact Microwave Microfluidic Sensor Using a Re-Entrant Cavity", Sensor, 19.03.2018 und der Druckschrift Prafull Sharma et al, "A microwave cavity resonator sensor for water-in-oil measurements", Sensors and Actuators B: Chemical, Volume 262, 1 June 2018, Seiten 200-210 werden Lösungen beschrieben, wie Feuchte in Ölen mithilfe von Hohlraumresonatoren bestimmt werden kann. Die Probleme einer Temperaturkompensation werden dabei nicht angesprochen.

Eine einfache und naheliegende Lösung wäre der temperaturstabilisierte Betrieb des Hohlraumresonators. Diese Maßnahme erfordert aber einen zusätzlichen technischen Aufwand und schränkt die Einsatzfähigkeit dieses Sensors ein.

### ZUSAMMENFASSUNG DER ERFINDUNG

Es ist Aufgabe der Erfindung, eine Lösung anzugeben, mit der die Änderung eines flüssigen Analyten einfach und über den gesamten Betriebstemperaurbereichs exakt bestimmt werden kann.

Die Erfindung ergibt sich aus den Merkmalen der unabhängigen Ansprüche. Vorteilhafte Weiterbildungen und Ausgestaltungen sind Gegenstand der abhängigen Ansprüche. Weitere Merkmale, Anwendungsmöglichkeiten und Vorteile der Erfindung ergeben sich aus der nachfolgenden Beschreibung.

Zur Charakterisierung des Analyten (auch mit MUT = Material Under Test bezeichnet), z.B. eines technischen Öls, wird durch eine Elektronik das RF-Spektrum, im Folgenden z.B. das Transmissionsspektrum (S21), in der Umgebung einer oder mehrerer Resonanzmoden des Hohlraumresonators und die zugehörige Temperatur gemessen. Dieses Spektrum ist charakteristisch für das MUT. Die Temperatur hat in der Regel einen spezifischen Einfluss auf die "Form des Spektrums"; es ergibt sich eine spezifische Signatur für die Temperatur, die sich von Änderungen der dielektrischen Eigenschaften des MUT, beispielsweise durch eine Feuchteaufnahme oder eine Alterung, unterscheidet.

Ein Aspekt der Erfindung besteht darin, die temperaturabhängige Charakteristik der Transmissionsspektren des "jungfräulichen" MUTs im Ausgangszustand vorab zu ermitteln und in einem Speicher zu hinterlegen. Dabei kann es sich um im spezifizierten Temperaturbereich gemessene Spektren handeln. Es besteht aber auch die Möglichkeit, nur an wenigen Stützpunkten Spektren aufzunehmen und bei bekannter spezifischer Signatur der Temperatur im Spektrum die Spektren bei weiteren Temperaturen zu berechnen. Letzteres ist dann vor allem von Vorteil, wenn sich fertigungstechnisch die Transmissionsspektren der Resonatoren geringfügig unterscheiden. Diese temperaturabhängige Charakteristik kann für unterschiedliche Analyten aufgenommen werden, für die der Hohlraumresonator eingesetzt werden soll.

Mit jeder Messung wird nun das aufgenommene Spektrum mit dem dazugehörigen jungfräulichen Spektrum bei dieser Temperatur beispielsweise durch Bildung des Differenzspektrums verglichen. Bei einem unveränderten Öl ergibt sich theoretisch eine Nullabweichung im gesamten Spektrum. In der Praxis wird es immer Abweichungen geben, z.B. als Folge von Alterung und Verunreinigungen. Auch die Aufnahme von Feuchte wird zu einer Abweichung führen. Entsprechend ist ein Schwellwert festzulegen, über dem Abweichungen nicht mehr toleriert werden und eine Warnung über den Ölzustand ausgegeben wird. Ist die Signatur der Störung (z.B. Feuchte) charakteristisch und bekannt, lässt sich aus der Form der Differenzkurve z.B. auch mit Unterstützung einer multivariaten Datenanalyse qualitativ und quantitativ auf die Ursache rückschließen.

Der Vorteil der Erfindung besteht darin, dass keine Temperaturstabilisierung für den Betrieb des Ölüberwachungssystems notwendig ist. Auch entfällt der klassische Ansatz der Verwendung eines Referenzsensors, welcher ebenfalls einer zeitlichen Alterung der MUTs ausgesetzt und somit nur bedingt tauglich wäre. Ein weiterer Vorteil ist, dass fertigungstechnisch bedingte geringe Unterschiede in der RF-Charakteristik der Resonatoren kompensiert werden. Das System kann für verschiedene MUTs. z.B. Ölsorten eingesetzt werden, es müssen lediglich die temperaturabhängige Charakteristik der Transmissionsspektren der jungfräulichen Probe im System hinterlegt sein. Durch die Aufnahme von Spektren (nicht nur charakteristische Punkte wie Resonanzfrequenz) lässt sich unter der Voraussetzung einer spezifischen Signatur eines Fehlers im Spektrum über die Störungsmeldung hinaus eine Aussage über die mögliche Fehlerursache treffen.

Die Erfindung beansprucht ein automatisiertes Verfahren zur Bestimmung der Veränderung eines flüssigen Stoffs (= MUT) - beispielsweise ein Öl - zwischen einem ersten Zeitpunkt und einem zweiten Zeitpunkt bei einer gemessenen Betriebstemperatur, wobei ein repräsentativer Teil eines Transmissionsspektrums des in einem Mikrowellen Hohlraumresonator eingebrachten Stoffs zu dem zweiten Zeitpunkt ermittelt wird und wobei der ermittelte repräsentative Teil des Transmissionsspektrums mit dem vorab zu dem ersten Zeitpunkt bei der gleichen Betriebstemperatur ermittelten repräsentativen Teil eines Referenz-Transmissionsspektrums eines gleichen, aber unberührten Stoffs verglichen wird und wobei eine Abweichung der beiden repräsentativen Teile voneinander ermittelt wird.

Ein unberührter Stoff ist ein "jungfräulicher Stoff", d.h. ein Stoff, der noch keiner Veränderung unterzogen wurde und/oder der nicht in einem Betriebseinsatz war, also noch keine Veränderung zeigen kann. Der erste Zeitpunkt liegt früher als der zweite Zeitpunkt.

In einer Weiterbildung wird der vorab zu dem ersten Zeitpunkt bei der gleichen Betriebstemperatur ermittelte repräsentative Teil des Referenz-Transmissionsspektrums durch Interpolation des Referenz-Transmissionsspektrums bei benachbarten Temperaturen ermittelt. Dadurch ist es nicht notwendig, Referenz-Transmissionsspektren bei allen möglichen Betriebstemperaturen aufzunehmen.

In einer weiteren Ausführungsform wird die Abweichung vorzugsweise als Differenzkurve ermittelt.

In einer weiteren Ausprägung wird bei einer charakteristischen und bekannten Signatur einer Störung aus der Form der Differenzkurve qualitativ und quantitativ die Störung ermittelt. Eine Störung ist beispielsweise ein unerwünschter Feuchtegehalt in einem Öl, wobei aus der Veränderung auf den Feuchtegehalt geschlossen werden kann.

In einer weiteren Ausgestaltung wird zur Ermittlung der Störung eine multivariate Datenanalyse ausgeführt. Mit Hilfe von multivariaten Verfahren (auch als multivariate Analysemethoden bezeichnet) werden in der multivariaten Statistik mehrere statistische Variablen oder Zufallsvariablen zugleich untersucht. Beispielsweise können für Fahrzeuge die Variablen Anzahl der Sitze, Gewicht, Länge usw. erhoben werden. In der univariaten Analyse hingegen wird jede Variable einzeln analysiert. Zusammenhangs- bzw. Abhängigkeitsstrukturen zwischen den Variablen, z. B. größere Anzahl von Sitzen bedingt ein größeres Gewicht, können nur mit einer multivariaten, nicht aber mit einer univariaten Analyse erkannt werden.

Die Erfindung beansprucht auch eine Vorrichtung zur Bestimmung der Veränderung eines flüssigen Stoffs (= MUT) zwischen einem ersten Zeitpunkt und einem zweiten Zeitpunkt bei einer gemessenen Betriebstemperatur. Die Vorrichtung weist auf:
- einen Mikrowellen Hohlraumresonator, in dem der Stoff eingebracht ist, und
- eine Mess- und Recheneinheit, die eingerichtet ist,
   > einen repräsentativen Teil eines Transmissionsspektrums des in dem Mikrowellen Hohlraumresonator befindlichen Stoffs zu dem zweiten Zeitpunkt zu ermitteln,
   > den ermittelten repräsentativen Teil des Transmissionsspektrums mit dem vorab zu dem ersten Zeitpunkt bei der gleichen Betriebstemperatur ermittelten repräsentativen Teil eines Referenz-Transmissionsspektrums eines gleichen, aber unberührten Stoffs zu vergleichen und
   > eine Abweichung der beiden repräsentativen Teile voneinander zu ermitteln.

In einer Weiterbildung kann die Mess- und Recheneinheit eingerichtet sein, den vorab zu dem ersten Zeitpunkt bei der gleichen Betriebstemperatur ermittelten repräsentativen Teil des Referenz-Transmissionsspektrums durch Interpolation des Referenz-Transmissionsspektrums bei benachbarten Temperaturen zu ermitteln.

In weiteren Ausführungsform kann die Mess- und Recheneinheit eingerichtet sein, die Abweichung als Differenzkurve zu ermitteln, oder einer charakteristischen und bekannten Signatur einer Störung aus der Form der Differenzkurve qualitativ und quantitativ die Störung zu ermitteln.

In einer weiteren Ausgestaltung kann die Mess- und Recheneinheit eingerichtet sein, zur Ermittlung der Störung eine multivariate Datenanalyse auszuführen. Auch können Verfahren der Künstlicher Intelligenz zur Fehlererkennung herangezogen werden, z.B. Modelle auf Basis neuronaler Netze.

In einer Weiterbildung der Vorrichtung kann der flüssige Stoff ein Öl, insbesondere ein technisches Öl, wie zum Beispiel ein Schmieröl, sein.

Weitere Besonderheiten und Vorteile der Erfindung werden aus den nachfolgenden Erläuterungen eines Ausführungsbeispiels anhand von schematischen Zeichnungen ersichtlich.

### KURZE BESCHREIBUNG DER ZEICHNUNGEN

Es zeigen:
FIG. 1 ein Ablaufdiagramm eines automatisierten Verfahrens zur Bestimmung der Veränderung eines Öls und
FIG. 2 ein Blockschaltbild einer Vorrichtung zur Bestimmung der Veränderung eines Öls.

### DETAILLIERTE BESCHREIBUNG DER ERFINDUNG

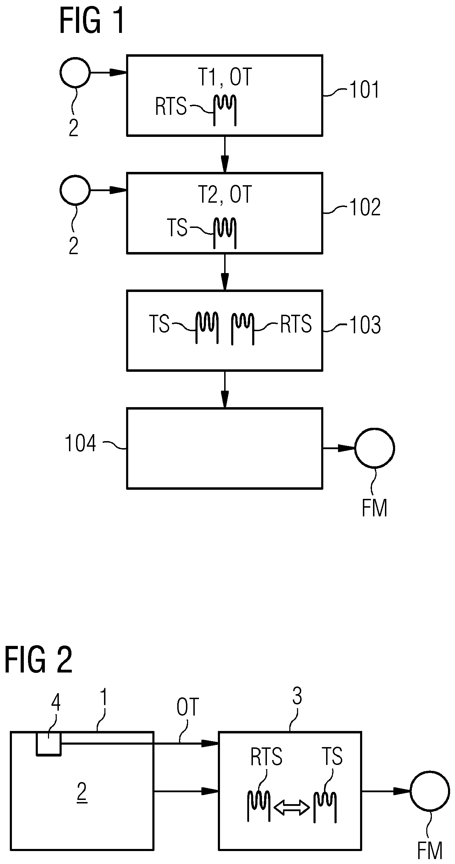

FIG. 1 zeigt ein Ablaufdiagramm eines automatisierten Verfahrens zur Bestimmung der Veränderung eines Öls 2, als Beispiel für einen flüssigen Stoff (=MUT).

In einem ersten Schritt 101 wird zu einem ersten Zeitpunkt T1, der vor einem zweiten Zeitpunkt T2 liegt, bei unterschiedlichen Betriebstemperaturen OT ein repräsentativer Teil von Referenz-Transmissionsspektren RTS des unberührten Öls 2, das zur Messung in einen Mikrowellen-Hohlraumresonator 1 eingebracht ist, mit Hilfe einer Mess- und Recheneinheit 3 ermittelt und in der Mess- und Recheneinheit 3 gespeichert. Der repräsentative Teil ist z.B. in der Umgebung einer oder mehrerer Resonanzmoden des Hohlraumresonators 1 vorhanden.

In einem zweiten Schritt 102 wird der gleiche repräsentative Teil eines Transmissionsspektrums TS des in dem Mikrowellen Hohlraumresonator 1 eingebrachten Öls 2 zu einem zweiten Zeitpunkt T2 ermittelt. Zu dem zweiten Zeitpunkt T2 hat das Öl 2 bereits eine Betriebszeit hinter sich und kann daher Veränderungen aufweisen.

In einem dritten Schritt 103 wird der ermittelte repräsentative Teil des Transmissionsspektrums T2 mit dem vorab zu dem ersten Zeitpunkt T1 bei der gleichen Betriebstemperatur OT ermittelten repräsentativen Teil des Referenz-Transmissionsspektrums RTS verglichen, wobei eine Abweichung der beiden repräsentativen Teile voneinander ermittelt wird.

In einem vierten Schritt 104 wird überprüft, ob die im dritten Schritt 103 ermittelte Abweichung signifikant ist, beispielsweise einen Schwellwert oder eine Schwellwertkurve überschreitet. Ist dies der Fall, gibt die Mess- und Recheneinheit 3 eine Fehlermeldung FM aus. Die Abweichung kann beispielsweise als Differenzkurve ermittelt werden.

Bei einer charakteristischen und bekannten Signatur einer Störung kann aus der Form der Differenzkurve qualitativ und quantitativ eine Störung, wie zum Beispiel ein Feuchtegehalt, ermittelt werden. Zur Ermittlung der Störung kann insbesondere eine multivariate Datenanalyse eingesetzt werden.

FIG. 2 zeigt ein Blockschaltbild einer Vorrichtung zur Bestimmung der Veränderung eines Öls 2 bei einer bestimmten Betriebstemperatur OT, zur Durchführung eines Verfahrens gemäß FIG. 1. Das Öl 2 ist ein typisches Beispiel für einen flüssigen Stoff (= MUT) .

Die Vorrichtung weist auf: Einen Mikrowellen Hohlraumresonator 1, in dem das Öl 2 eingebracht ist, und eine Mess- und Recheneinheit 3. Die Mess- und Recheneinheit 3 ist eingerichtet, einen repräsentativen Teil eines Transmissionsspektrums TS des in dem Mikrowellen Hohlraumresonator 1 befindlichen Öls 2 zu einem zweiten Zeitpunkt T2 zu ermitteln, den ermittelten repräsentativen Teil des Transmissionsspektrums TS mit dem vorab zu einem ersten Zeitpunkt T1 bei der gleichen Betriebstemperatur OT - gemessen mit einem Temperaturfühler 4 - ermittelten repräsentativen Teil eines Referenz-Transmissionsspektrums RTS eines gleichen, aber unberührten Öls 2 zu vergleichen und eine Abweichung der beiden repräsentativen Teile voneinander zu ermitteln. Ist die Abweichung signifikant, d.h. übersteigt sie einen vorgegebenen Schwellwert, wird durch die Mess- und Recheneinheit 3 eine Fehlermeldung FM ausgegeben.

Obwohl die Erfindung im Detail durch die Ausführungsbeispiele näher illustriert und beschrieben wurde, ist die Erfindung durch die offenbarten Beispiele nicht eingeschränkt und andere Variationen können vom Fachmann daraus abgeleitet werden, ohne den Schutzumfang der Erfindung zu verlassen.

### Bezugszeichenliste

- 1: Mikrowellen-Hohlraumresonator
- 2: Öl
- 3: Mess- und Recheneinheit
- 4: Temperaturfühler

- 101: erster Schritt
- 102: zweiter Schritt
- 103: dritter Schritt
- 104: vierter Schritt

- FM: Fehlermeldung
- OT: Betriebstemperatur
- RTS: Referenz-Transmissionsspektrum
- T1: erster Zeitpunkt
- T2: zweiter Zeitpunkt
- TS: Transmissionsspektrum

## Patentansprüche

1. Automatisiertes Verfahren zur Bestimmung der Veränderung eines flüssigen Stoffs (2) zwischen einem ersten Zeitpunkt (T1) und einem zweiten Zeitpunkt (T2) bei einer gemessenen Betriebstemperatur (OT),
- wobei ein repräsentativer Teil eines Transmissionsspektrums (TS) des in einem Mikrowellen Hohlraumresonator (1) eingebrachten Stoffs (2) zu dem zweiten Zeitpunkt (T2) ermittelt wird und
- wobei der ermittelte repräsentative Teil des Transmissionsspektrums (TS) mit dem vorab zu dem ersten Zeitpunkt (T1) bei der gleichen Betriebstemperatur (OT) ermittelten repräsentativen Teil eines Referenz-Transmissionsspektrums (RTS) eines gleichen, aber unberührten und/oder unveränderten Stoffs (2) verglichen wird und
- wobei eine Abweichung der beiden repräsentativen Teile voneinander ermittelt wird.

2. Verfahren nach Anspruch 1,
- wobei der vorab zu dem ersten Zeitpunkt (T1) bei der gleichen Betriebstemperatur (OT) ermittelte repräsentative Teil des Referenz-Transmissionsspektrums (RTS) durch Interpolation des Referenz-Transmissionsspektrums (RTS) bei benachbarten Temperaturen ermittelt wird.

3. Verfahren nach Anspruch 1 oder 2,
- wobei die Abweichung als Differenzkurve ermittelt wird.

4. Verfahren nach Anspruch 3,
- wobei bei einer charakteristischen und bekannten Signatur einer Störung aus der Form der Differenzkurve qualitativ und quantitativ die Störung ermittelt wird.

5. Verfahren nach Anspruch 4,
- wobei zur Ermittlung der Störung eine multivariate Datenanalyse ausgeführt wird.

6. Verfahren nach einem der vorhergehenden Ansprüche, wobei der flüssige Stoff ein Öl (2) ist.

7. Vorrichtung zur Bestimmung der Veränderung eines flüssigen Stoffs (2) zwischen einem ersten Zeitpunkt (T1) und einem zweiten Zeitpunkt (T2) bei einer gemessenen Betriebstemperatur (OT), aufweisend:
- einen Mikrowellen Hohlraumresonator (1), in dem der flüssige Stoff (2) eingebracht ist, und
- eine Mess- und Recheneinheit (3), die eingerichtet ist,
> einen repräsentativen Teil eines Transmissionsspektrums (TS) des in dem Mikrowellen Hohlraumresonator (1) befindlichen flüssigen Stoffs (2) zu dem zweiten Zeitpunkt (T2) zu ermitteln,
> den ermittelten repräsentativen Teil des Transmissionsspektrums (OT) mit dem vorab zu dem ersten Zeitpunkt (T1) bei der gleichen Betriebstemperatur (OT) ermittelten repräsentativen Teil eines Referenz-Transmissionsspektrums (RTS) eines gleichen, aber unberührten Stoffs (2) zu vergleichen und
> eine Abweichung der beiden repräsentativen Teile voneinander zu ermitteln.

8. Vorrichtung nach Anspruch 7, **dadurch gekennzeichnet, dass** die Mess- und Recheneinheit (3) eingerichtet ist, den vorab zu dem ersten Zeitpunkt (T1) bei der gleichen Betriebstemperatur (OT) ermittelten repräsentativen Teil des Referenz-Transmissionsspektrums (RTS) durch Interpolation des Referenz-Transmissionsspektrums (RTS) bei benachbarten Temperaturen zu ermitteln.

9. Vorrichtung nach Anspruch 7 oder 8, **dadurch gekennzeichnet,**
**dass** die Mess- und Recheneinheit (3) eingerichtet ist, die Abweichung als Differenzkurve zu ermitteln.

10. Vorrichtung nach Anspruch 9, **dadurch gekennzeichnet, dass** die Mess- und Recheneinheit (3) eingerichtet ist, bei einer charakteristischen und bekannten Signatur einer Störung aus der Form der Differenzkurve qualitativ und quantitativ die Störung zu ermitteln.

11. Vorrichtung nach Anspruch 10, **dadurch gekennzeichnet, dass** die Mess- und Recheneinheit (3) eingerichtet ist, zur Ermittlung der Störung eine multivariate Datenanalyse auszuführen.

12. Vorrichtung nach einem der Ansprüche 7 bis 11, **dadurch gekennzeichnet,**
**dass** der Stoff ein technisches Öl (2), insbesondere ein Schmieröl, ist.
